# EUROPEAN PATENT APPLICATION

(11) **EP 4 108 251 A1**
(43) Date of publication of application: **28.12.2022**
(21) Application number: 21180824.1
(22) Date of filing: 22.06.2021
(51) Int. Cl.: A61K 38/16, A61K 47/54, A61K 9/127, A61K 47/69, A61P 31/14, A61P 31/16

(54) **GRIFFITHSIN FOR USE IN A METHOD OF PREVENTING OR TREATING INFECTIONS WITH RESPIRATORY VIRUSES**

(71) Applicant: Solmic Biotech GmbH, 40225 Düsseldorf (DE)
(72) Inventor: FURCH, Marcus, 65719 Hofheim (DE); BESECKE, Karen F. W., 30167 Hannover (DE); PANNEM, Rajeswara Rao, 69168 Wiesloch (DE)
(74) Representative: Schüssler, Andrea

(57) **Abstract**

The present invention relates to Griffithsin (GRFT) for use in a method of preventing or treating infections with respiratory viruses, wherein the GRFT is encapsulated. A preferred embodiment is a combination of targeted nanocarrier-mediated delivery of GRFT and non-encapsulated GRFT to CLR-positive cells infected with, or susceptible to infection with, a respiratory virus.

## Description

### 1. FIELD OF THE INVENTION

The present invention relates to Griffithsin (GRFT) for use in a method of preventing or treating infections with respiratory viruses, wherein the GRFT is encapsulated. A preferred embodiment is a combination of targeted nanocarrier-mediated delivery of GRFT and non-encapsulated GRFT to CLR-positive cells or CRD-positive cells infected with, or susceptible to infection with, a respiratory virus.

### 2. DISCUSSION OF THE RELATED ART

Many viruses that enter the patient's body via the respiratory tract have the potential to cause pandemic situations. One example that had been underestimated at the beginning is coronavirus disease 2019 (COVID-19) that is caused by a novel coronavirus called SARS-CoV-2. The acronym "SARS" stands for "severe acute respiratory syndrome". An outbreak of COVID-19 in the Hubei province (China) at the end of 2019 was spreading globally and is still impacting the health of people and of the economy worldwide. As of May 30, 2021, COVID-19 has been confirmed in about 171 million people worldwide with over 3.5 million deaths, i.e. carrying a mortality of approximately 2.3%. This is an example for a worldwide pandemic but there were already candidates in the past that had the (theoretical) capability to cause similar damage which fortunately did not happen, e.g. influenza virus A/H1N1 (porcine flu), influenza virus A/H5N1(avian flu), coronavirus SARS-CoV-1 or coronavirus MERS-CoV (Middle East Respiratory Syndrome).

Most of these viruses cause symptoms such as dry cough, fever, headache, a runny nose and fatigue. The virus spreads from person to person. Droplet infection is the main mode of transmission. Transmission can take place directly, from person-to-person, or indirectly through contact between hands and the mucous membranes of the mouth, the nose or the conjunctiva of the eyes. There have been reports of persons who were infected by individuals who had only shown slight or non-specific symptoms of disease.

Unlike other acute infectious diseases progressing to sepsis, the severe courses of COVID-19 seemingly show prolonged progression from onset of first symptoms to life-threatening deterioration of (primarily) lung function including difficulty in breathing and pneumonia. Severe acute respiratory distress syndrome (ARDS) reflects the hallmark of a critical course of the disease. Treatment of the infection depends on the severity of the disease presentation (e.g. administering oxygen, maintaining fluid balance, if necessary administering antibiotics to combat bacterial co-infections) and also includes the treatment of relevant underlying chronic illnesses. Current management of COVID-19 is supportive, and ARDS is the leading cause of mortality. There are also many cases of long-COVID. This means that the patients have no detectable virus load any longer but do not recover from the symptoms (e.g. difficulty to breathe, fatigue, joint pain) over months.

There is an urgent need for an effective prevention or treatment of viral infections of the respiratory tract or for the avoidance of severe courses of these diseases. For COVID-19 the current focus has been on the development of novel therapeutics, including antiviral agents and vaccines. The first vaccines have been approved in the 4^{th} quarter of 2020. However, there is still not enough vaccine available to satisfy the worldwide demands. Thus, the pandemic will still proceed and there is an urgent need for the prevention and/or treatment of COVID-19 using existing, approved therapies with proven safety profiles to address the immediate need to reduce the rising mortality. One of such known drugs with a good safety profile is Griffithsin (GRFT). It has been reported as a highly potent broad-spectrum antiviral lectin for the treatment of HIV (Lee, C., Mar. Drugs 2019, 17:567) and viruses of the family coronaviridae (J. of Virology, 2010, Vol. 84, No. 5, pp. 2511-2521).

GRFT is a carbohydrate-binding protein made of 121 amino acids and is 12.7 kDa in size. It has an unique amino acid residue at position 31 which does not match any of the 20 standard amino acids. It was initially isolated from an aqueous extract of the red algae *Griffithsia sp.* collected from waters off the shores of New Zealand. Researchers at the U.S. National Cancer Institute first reported its potent cytoprotective activity against HIV-1 in T-lymphoblastoid cells. In terms of structural classification, GRFT is a Jacalin-related lectin harboring three repeats of an antiparallel four-stranded β-sheet with a triangular prism shape. It is called a domain-swapped dimer because two β-strands from one protein forming the dimer display domain-exchanging properties with the same two β-strands of its counterpart. It has three identical carbohydrate-binding sites (located within residues 20-34, 58-76, and 96-120) on each monomer with three conserved glycine-rich repeats (GGSGG) (Lee, C., Mar. Drugs 2019, 17:567).

### 3. SUMMARY OF THE INVENTION

The inventors have found out that nanoencapsulated GRFT has advantages as a prophylactic or therapeutic option for infections caused by a respiratory virus compared to free (non-encapsulated) GRFT, in particular for coronavirus infections caused by SARS-CoV-2 or infections with influenza virus.

In general, cell-targeted nanocarriers are a suitable vehicle for specifically delivering encapsulated compounds to (a) given cell type(s) expressing the respective targeting structure. When employed therapeutically, such technology highly focuses in delivering compound to the cell type(s) of choice. As an example, it has been shown earlier that the compounds needed to be delivered by targeted liposomes selectively reached the cell type of choice, such as myeloid dendritic cells (mDCs), and delivered their contents into such cells (Gieseler RK et al., Scand J Immunol. 2004; 59:415-24; WO 2005/092288 A1).

Thus, the present invention concerns an active agent complex comprising GRFT encapsulated in a lipid-based or polymer-based nanocarrier and at least one cell-specific targeting ligand on the surface of the nanocarrier, wherein the targeting ligand specifically binds to a receptor on the surface of a carbohydrate recognition domain (CRD)-positive cell infected with, or susceptible to infection with, a respiratory virus for use in a method of preventing or treating an infection caused by respiratory viruses, in particular influenza viruses or corona viruses.

In a particular preferred embodiment the present invention concerns a pharmaceutical combination comprising
(a) an active agent complex comprising GRFT encapsulated in a lipid-based or polymer-based nanocarrier and at least one cell-specific targeting ligand on the outer surface of the nanocarrier, wherein the targeting ligand specifically binds to a receptor on the surface of a CRD-positive cell or CLR-positive cell infected with, or susceptible to infection with, a respiratory virus, and
(b) non-encapsulated GRFT
for use in a method of preventing or treating an infection caused by respiratory viruses, in particular of a coronavirus infection or influenza virus infection.

The GRFT is as the active agent part of an agent complex that is furnished on its outer surface with a targeting ligand, specifically recognizing C-type lectin receptor (CLRs) or carbohydrate recognition domains (CRDs), and enabling intracellular uptake. Targeting systems as disclosed here are comprised of nanocarriers that feature targeting ligands directly embedded in their outer surfaces; alternatively, such targeting ligands may be positioned externally on a spacer embedded in the outer surface of the nanocarriers, offering desired additional features. Optionally, targeting systems may further be supplemented with agents facilitating the intracellular (i.e. specifically, endosomal) release of encapsulated active agents.

### 4. BRIEF DESCRIPTION OF THE FIGURES

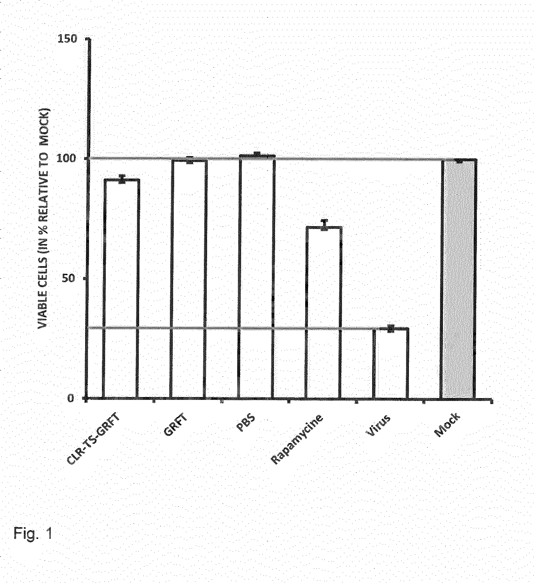

Fig. 1: Effect of encapsulated GRFT formulations in VeroE6 cell viability
   Cells were treated with CLR-TargoSphere^{®} encapsulated GRFT formulations (0.5 µM, dialysed), unencapsulated GRFT (0.5 µM), phosphate-buffered saline that was used for dilution of GRFT (PBS, 0.5 µM) and Rapamycine (10 µM). At the same time for a positive control, cells were infected with SARS-CoV-2 strain Muc-IMB-1 with multiplicity of infection (MOI) 0.1. For mock control, cells were neither infected nor treated. Cell viability measured after 3 days of post infection or treatment. The upper line indicates 100% viable cells in mock control and the lower line shows cell viability in virus infected positive control as measured in MTS assay.

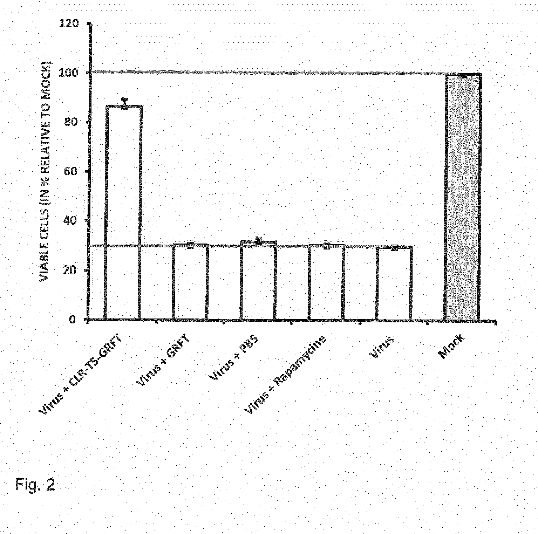

Fig. 2: Effect of CLR-TargoSphere encapsulated GRFT formulations in SARS-CoV-2 strain Muc-IMB-1 infected VeroE6 cell viability
   VeroE6 cells were infected with SARS-CoV-2 strain Muc-IMB-1 and treated simultaneously with CLR-TargoSphere^{®} encapsulated GRFT formulation (0.5 µM, dialysed), unencapsulated GRFT (0.5 µM), phosphate-buffered saline that was used for dilution of GRFT (PBS, 0.5 µM) and Rapamycine (10 µM) as shown in the figure. At the same time for a positive control, cells were only infected with SARS-CoV-2 strain Muc-IMB-1. For mock control, cells were neither infected nor treated. Cell viability measured after 3 days of post infection and/or treatment. The upper line indicates 100% viable cells in mock control and the lower line shows cell viability in virus infected positive control as measured in MTS assay.

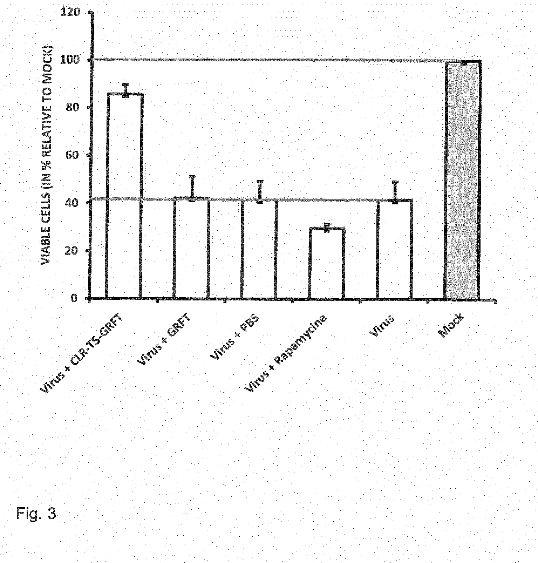

Fig. 3: Effect of CLR-TargoSphere^{®} encapsulated GRFT formulations in SARS-CoV-2 strain Muc-IMB-CB infected VeroE6 cell viability
   VeroE6 cells were infected with SARS-CoV-2 strain Muc-IMB-CB and treated simultaneously with CLR-TargoSphere encapsulated GRFT formulation (0.5 µM, dialysed), unencapsulated GRFT (0.5 µM), phosphate-buffered saline that was used for dilution of GRFT (PBS, 0.5 µM) and Rapamycine (10 µM) as shown in the figure. At the same time for a positive control, cells were only infected with SARS-CoV-2 strain Muc-IMB-CB virus. For mock control, cells were neither infected nor treated. Cell viability measured after 3 days of post infection and/or treatment. The upper line indicates 100% viable cells in mock control and the lower line shows cell viability in virus infected positive control as measured in MTS assay.

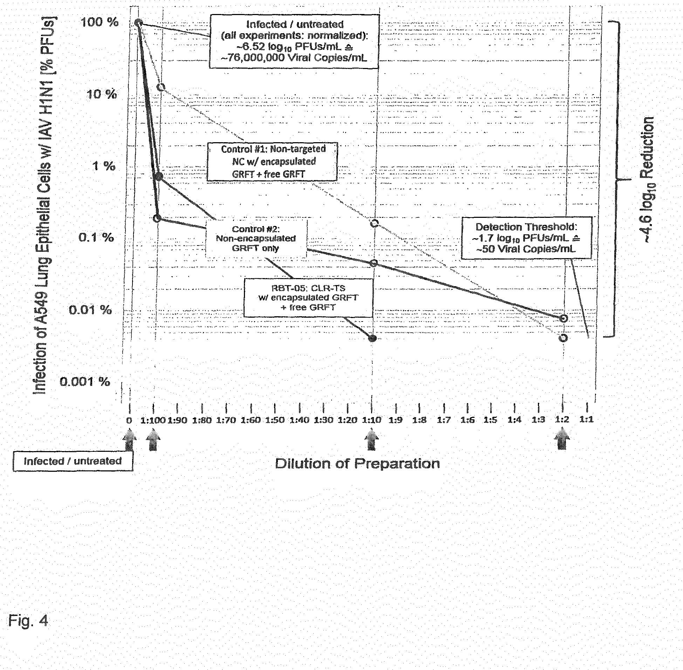

Fig. 4: Efficacy of TS(FC4C-NC)-delivered Griffithsin (GRFT) + free GRFT against intracellular H1N1 infection
   In the infection model employing the host cells and the IAV H1N1v strain, intracellular inhibition of viral propagation by RBT-05 (CLR-TS w/ GRFT + free GRFT), by non-targeted nanocarrier encapsulated plus free GRFT, and by non-encapsulated GRFT was tested in serial dilutions of 1:2 to 1:100 against untreated infection conditions. The read-out was performed by determining virus-induced plaque-forming units (PFUs) relating to the actual viral copy numbers (n = 3 per reading point). Data were normalized and set at 100% for the non-treated infection control

### 5. DETAILED DESCRIPTION OF THE PREFERRED EMBODIMENTS

Thus, the present invention concerns an active agent complex comprising GRFT encapsulated in a lipid-based or polymer-based nanocarrier and at least one cell-specific targeting ligand on the surface of the nanocarrier, wherein the targeting ligand specifically binds to a receptor on the surface of a CRD-positive cell or CLR-positive cell infected with, or susceptible to infection with, a respiratory virus for use in a method of preventing or treating an infection caused by respiratory viruses, in particular influenza virus or coronavirus.

In a preferred embodiment the present invention concerns a lipid-drug complex comprising GRFT encapsulated in a liposome and at least one carbohydrate (e.g. monofucose, polyfucose) or derivative thereof as a targeting ligand on the outer surface of the liposome, wherein the targeting ligand specifically binds to a receptor expressing C-type lectin-like carbohydrate recognition domains (CRD) or other receptors with CRD on the surface of a cell infected with, or susceptible to infection with, a respiratory virus, in particular influenza virus or coronavirus.

In a preferred embodiment the present invention concerns a pharmaceutical combination comprising
(a) an active agent complex comprising GRFT encapsulated in a lipid-based or polymer-based nanocarrier and at least one cell-specific targeting ligand on the outer surface of the nanocarrier, wherein the targeting ligand specifically binds to a receptor on the surface of a CLR-positive cell or CRD-positive cell infected with, or susceptible to infection with, a respiratory virus and
(b) non-encapsulated GRFT
for use in a method of preventing or treating an infection caused by respiratory viruses, e.g. infections by influenza virus or coronavirus.

In a particular preferred embodiment, the present invention concerns a pharmaceutical combination comprising
(a) a lipid-drug complex comprising GRFT encapsulated in a liposome and at least one carbohydrate (e.g. monofucose, polyfucose) or derivative thereof as a targeting ligand on the outer surface of the liposome, wherein the targeting ligand specifically binds to a receptor expressing C-type lectin-like carbohydrate recognition domains or other receptors with carbohydrate recognition domains on the surface of a cell infected with, or susceptible to infection with, a respiratory virus, and
(b) non-encapsulated GRFT
for use in a method of treating or preventing of an infection with respiratory viruses, in particular coronavirus or influenza virus.

In a preferred embodiment the active agent complex is part of a pharmaceutical composition or combination combined with a pharmaceutically acceptable carrier.

The present invention enables the preferentially delivering of GRFT as an active agent to a CLR- positive or CRD-positive cell of a mammalian subject, including a human.

In the present application the terms "nanocarrier", "active agent complex", "active agent formulation" and "active agent system" are used interchangeably and are used synonymously to each other.

Targeted CRD-positive cells or CLR-positive cells (both terms are used interchangeably) include, but are not limited to, airway epithelial cells (e.g. lung vascular cells), endothelial cells of the lymphatic or blood system, and immune cells (e.g. bone marrow-resident myeloid progenitor cells, monocytes, myeloid dendritic cells, follicular dendritic cells, plasmacytoid dendritic cells, macrophages (e.g. alveolar macrophages), T lymphocytes, and natural killer cells). In the following, the term "dendritic cell" includes a myeloid, a plasmacytoid, or a follicular dendritic cell unless not stated otherwise. The term "T lymphocyte" includes, but is not limited to, a T-helper, a T-regulatory, or a T-memory cell.

In a particular preferred embodiment the present invention is directed to target CRD-positive cells or CLR-positive cells of the respiratory tract. The respiratory tract is the subdivision of the respiratory system involved with the process of respiration in mammals. The respiratory tract is lined with respiratory mucosa or respiratory epithelium (also called airway epithelial cells). The respiratory tract is divided into the upper airways and lower airways. The upper airways or upper respiratory tract includes the nose and nasal passages, paranasal sinuses, the pharynx, and the portion of the larynx above the vocal folds (cords). The lower airways or lower respiratory tract includes the portion of the larynx below the vocal folds, trachea, bronchi and bronchioles. The lungs can be included in the lower respiratory tract or as separate entity and include the respiratory bronchioles, alveolar ducts, alveolar sacs, and alveoli.

The CRD-positive cell or CLR-positive cell is infected with, or susceptible to infection with, a respiratory virus. Examples of such viruses that enter the body via the respiratory tract (= "respiratory viruses") and are causing also disease symptoms in the respiratory tract are human respiratory syncytial virus or human orthopneumovirus (including RSV A, RSV B, RSV S2, RSS2), influenza virus (e.g. influenza A, B, C and mixed human-animal types like A/H1N1, A/H7N9 or A/H5N1), parainfluenza viruses, metapneumovirus, rhinovirus, adenoviruses, bocaviruses or coronavirus, in particular SARS-CoV-1, MERS-CoV and SARS-CoV-2. It needs to be considered that some of these respiratory viruses can cause endemic, epidemic and/or pandemic situations.

According to the present invention the term "coronavirus" comprises both wild-type and mutants or variants. In case of SARS-CoV-2 it encompasses the "original" type of 2019 but also all later identified mutants or variants, like B.1.1.7 ("British variant", Alpha variant), B.1.351 ("South African variant", Beta variant), P.1 ("Brazilian variant", Gamma variant) or B.1.617 ("Indian variant", Delta variant). Of course, all forthcoming variants will be encompassed, too.

"Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at an effective dose. Additional pharmaceutically compatible carriers can include gels, bioadsorbable matrix materials, implantation elements containing the therapeutic agent, or any other suitable vehicle, delivery or dispensing means or material(s).

A "complex" or "formulation" is a mixture or adduct resulting from chemical binding or bonding between and/or among its constituents or components, including the lipid, active agent, and other optional components. Chemical binding or bonding can have the nature of a covalent, ionic, hydrogen, van der Waal's, or hydrophobic bond, or any combination of these bonding types linking the constituents of the complex at any of their parts or moieties, of which a constituent can have one or a multiplicity of moieties of various sorts. Not every constituent of a complex or formulation needs to be bound to every other constituent, but each constituent has at least one chemical binding or bonding with at least one other constituent of the complex. In accordance with the present invention, examples of lipid-active agent complexes include liposomes (lipid vesicles), or lipid-active agent sheet-disk complexes. Also, lipid-conjugated active agents can be a part of the lipid-active agent complex.

Useful techniques for making lipid-active agent formulations, such as liposomes, are known in the art (e.g., Sullivan SM et al., Antisense Res Dev 1992;2:187-97; Laverman P et al., Crit Rev Ther Durg Carrier Syst 2001;18:551-66); Oussoren C, Storm G, Adv Drug Deliv Rev 2001;50:143-56; U.S. Patent No. 5,773,027; U.S. Patent No. 5,223,263; WO 96/10399 A1).

For cellular targeting, in accordance with the present invention, liposomes or lipid-based nanocarriers can be prepared by any of the methods described above. They contain lipids that crosslink with an active agent, for example, protein . Examples of such lipids are N-glutaryl-phosphatidylethanolamine, *N*-succinyl-phosphatidylethanolamine, maleimidophenyl-utyryl-phosphatidylethanolamine,succinimidyl-acetylthioacetate-phosphatidylethanolamine, and SPDP-phosphatidylethanolamine. The glutaryl and succinimidyl phosphatidylethanolamine can be linked to a nucleophile (e.g. an amine) using cyclocarbodiimide. The maleimido, acetylthioacetate and SPDP phosphatidylethanolamines can be reacted with thiols on the proteins, peptides or small molecular weight ligands (<1000 g/mol). The protein can be derivatized to the liposomes after formation. Non-derivatized protein can be removed by gel permeation chromatography. Peptides and low molecular weight ligands can be derivatized to the lipids and added to the organic lipid solution prior to formulating the lipid film.

In accordance with the present invention, examples of useful lipids include any vesicle-forming lipid, such as, but not limited to, phospholipids, such as phosphatidylcholine (PC), both naturally occurring and synthetically prepared, phosphatidic acid (PA), lysophosphatidylcholine, phosphatidylserine (PS), phosphatidylethanolamine (PE), sphingolipids, phosphatidylglycerol (PG), sphingomyelin, cardiolipin, glycolipids, gangliosides, cerebrosides and the like used either singularly or intermixed such as in soybean phospholipids (e.g., Asolectin, Associated Concentrates). The PC, PG, PA and PE can be derived from purified egg yolk and its hydrogenated derivatives.

Optionally, other lipids such as steroids, cholesterol, aliphatic amines (e.g. long-chained aliphatic amines or carboxylic acids), long-chained sulfates and phosphates, diacetyl phosphate, butylated hydroxytoluene, tocopherols, retinols, and isoprenoid compounds can be intermixed with the phospholipid components to confer certain desired and known properties on the formed vesicles. In addition, synthetic phospholipids containing either altered aliphatic portions such as hydroxyl groups, branched carbon chains, cyclo-derivatives, aromatic derivatives, ethers, amides, polyunsaturated derivatives, halogenated derivatives, or altered hydrophilic portions containing carbohydrate, glycol, phosphate, phosphonate, quaternary amine, sulfate, sulfonate, carboxy, amine, sulfhydryl, or imidazole groups and combinations of such groups can be either substituted or intermixed with the above-mentioned phospholipids and used in accordance with the invention.

Saturated synthetic PC and PG, such as dipalmitoyl can also be used. Other amphipathic lipids used advantageously with PC are, e.g., gangliosides, globosides, fatty acids, stearylamine, and long-chain alcohols. PEGylated lipids, monoglycerides, diglycerides, and triglycerides can also be included. Acylated and diacylated phospholipids are also useful.

In some embodiments, useful phospholipids include egg phosphatidylcholine (EPC), dilauryloylphosphatidylcholine (DLPC), dimyristoylphosphatidylcholine (DOPC), dipalmitoylphosphatidylcholine (DPPC), distearoylphosphatidylcholine (DSPC), 1-myristoyl-2-palmitoylphosphatidylcholine (MPPC), 1-palmitoyl-2-myristoyl phosphatidylcholine (PMPC), 1-palmitoyl-2-stearoyl phosphatidylcholine (PSPC), 1-stearoyl-2-palmitoyl-phosphatidylcholine (SPPC), dioleoylphosphatidylycholine (DOPC), dilauryloylphosphatidylglycerol (DLPG), dimyristoylphosphatidylglycerol (DMPG), dipalmitoylphosphatidylglycerol (DPPG), distearoylphosphatidylglycerol (DSPG), distearoylsphingomyelin (DSSP), distearoylphophatidylethanolamine (DSPE), dioleoylphosphatidylglycerol (DOPG), dimyristoyl phosphatidic acid (DMPA), dipalmitoyl phosphatidic acid (DPPA), dimyristoyl phosphatidylethanolamine (DMPE), dipalmitoylphosphatidylethanolamine (DPPE), dimyristoyl phosphatidylserine (DMPS), dipalmitoylphosphatidylserine (DPPS), brain phosphatidylserine (BPS), brain sphingomyelin (BSP), and dipalmitoyl sphingomyelin (DPSP).

Useful polymers for preparing polymer-based nanocarriers are poly(vinyl pyridine), Poly(methyl methacrylate) (PMMA), Polystyrene, Polycarbonate, Poly(dimethyl siloxane), Poly(propylene carbonate), Polyimide, Poly(vinyl chloride), Poly(butylene terephthalate), Poly(lactic acid) (PLGA), Poly(lactide-co-glycolide-co-PEG) (PLGAePEG), Poly(ε-caprolactone), Polysaccharides like alginate and chitosan, Poly(butyl cyanoacrylate).

In one embodiment, PC and cholesterol are employed. However, any suitable molar ratio of a non-steroidal lipid:steroidal lipid (e.g., cholesterol) mixture can optionally be employed to promote the stability of a particular lipid-active agent complex during storage and/or delivery to a mammalian subject.

Active agent and lipids can be mixed by any useful known technique, e.g. sonication, vortexing, extrusion, microfluidization, homogenization, or use of a detergent (later removed, e.g., by dialysis).

For some active agents, the use of an organic solvent can facilitate the production of the lipid-active agent formulation. After the mixing the active agent and lipids, the organic solvent is removed by, e.g., evaporating by vacuum, application of heat, or hot ethanol injection (e.g., Deamer, United States Patent No. 4,515,736), as long as the lipid and active agent are stable at the temperature used. Dialysis and/or chromatography (e.g. affinity chromatography) can also be employed to remove the organic solvent. Hydrating the active agent is performed with water or any biocompatible aqueous buffer (e.g., phosphate-buffered saline, HEPES, or TRIS) that maintains physiological osmolarity. Rehydration of liposomes can be accomplished simultaneous to remove the organic solvent, or alternatively, can be delayed until a more convenient time for using the liposomes (e.g., Papahadjopoulos et al., United States Patent No. 4,235,871). The shelf-life of hydratable ("dry") liposomes - typically 8-12 months - can be increased by lyophilization.

In one embodiment of the present invention, the lipid-active agent complex is a unilamellar liposome. Such liposomes accommodate higher levels of active agent which may interact with inner and outer surfaces of the liposomes. However, in accordance with the present invention multilamellar liposomes and/or PEGylated liposomes can also be used.

The lipid-active agent complex is preferably, but not necessarily,∼30-200 nm in diameter.

In accordance with the present invention, lipid-active agent complexes can be preserved by any known method, such as lyophilization (e.g., Crowe et al., United States Patent No. 4,857,319). Typically, lyophilization or other useful cryopreservation techniques involve the inclusion of a cryopreservant, such as a disaccharide.

The active agent complex, pharmaceutical composition or combination of the present invention is administered to a subject by any suitable means, for example by injection, i.e. intravenous, subcutaneous, intraocular, intraarterial, intramuscular, intradermal or intranasal. Subcutaneous or intramuscular injection are preferred for introducing the lipid-active agent complex into lymphatic vessels. The lipid-active agent formulation can also be applied intrapulmonary by inhalation or intranasal application. This mode of administration is particularly preferred for an enrichment of the active agent complex in the lung. In this regard reference is made to Example 6.

In accordance with the present invention, "lymphoid tissue" is (i) a lymph node (e.g., inguinal, mesenteric, ileocecal, or axillary); (ii) the spleen; (iii) the thymus, (iv) the mucosa-associated lymphoid tissue such (e.g., in lung, intestinal lamina propria, or Peyer's patches of the small intestine), (v) the skin-associated lymphoid tissue, or (vi) Waldeyer's neck ring also encompassing the lingual, palatine and pharyngeal tonsils as anatomically discrete structures.

Injection is by any method that drains directly, or preferentially, into the lymphatic system or bone marrow but also to the blood stream. Most preferred are subcutaneous, intracutaneous, and bone marrow-directed injection routes employing a syringe needle gauge larger than the lipid-active agent formulation. Typically, the injection (generally ∼1-5 cm³) is into the subject's arm, leg, or belly, but any convenient site can be chosen for subcutaneous injection. In accordance with some embodiments of the present invention, when administered subcutaneously the active agent enters the lymphatic system prior to entering systemic blood circulation. Benefits include (i) its distribution throughout the lymphoid system and localization in lymph nodes; (ii) to avoid or minimize (serum) protein-mediated destabilization of lipid-active agent complexes; and (iii) the delivery of the agent at concentrations that cannot be achieved with a soluble form of the active agent administered by any other route of administration.

The frequency of administration is preferably two or three times per day, once per day, one to three times per week, but more or less frequent injections (e.g., monthly) can be appropriate, too.

In the pharmaceutical combination of the present invention parts (a) and (b) may be administered simultaneously or sequentially, wherein, preferably, (a) and (b) are administered simultaneously. Parts (a) and (b) may be provided in a single unit dosage form for being taken together or as separate entities (e.g. in separate containers) to be administered simultaneously or with a certain time difference. This time difference may be between 1 hour and 12 hours. Each combination part may be administered either alone or in a medicament which comprises the active agent complex and one or more pharmaceutically acceptable carriers, excipients and diluents, as defined above and according to standard pharmaceutical practice.

The targeting ligand can be a small chemical compound, an antibody, a sugar, a peptide or an aptamer. As used herein, the term "antibody"(Ab) is meant to include intact immunoglobulin molecules as well as antibody fragments (such as, for example, Fab and F(ab')2 fragments) which are capable of specifically binding to a surface receptor. Fab and F(ab')2 fragments lack the Fc fragment of intact antibody. Moreover, antibodies useful for the purposes of the present invention include chimeric, single chain, multifunctional (e.g. bispecific) and humanized antibodies or human antibodies.

The present invention uses nanocarriers, particularly liposomes, having targeting ligands on their inner and/or outer surface. These targeting ligands provide a surface labeling of the nanocarrier. For purposes of the present invention the targeting ligand is attached to the lipid component (e.g. a liposome) by techniques known in the art (WO 05/092288 A1) Preferred examples of targeting ligands are monofucose, polyfucose or derivatives thereof. One particular preferred targeting ligand is cholesten-5-yloxy-N-(4-((1-imino-2-D-thiofucosylethyl)amino)alkyl)formamide (Fuc-C4-chol), cholesteryl-(4-(3-((2R,3S,4R,5S,6S)-3,4,5-trihydroxy-6-(hydroxymethyl)tetrahydro-2H-pyran-2-yl)thioureido)butyl)carbamate (Thiourea-Galac-C4-chol). In a preferred embodiment, fucosylated (Fuc) liposomes are prepared by means of a linkage of fucosyl targeting ligand, may be to a spacer that is attached to a suitable lipophilic membrane anchor on the liposomes or may be to a distal end of the anchor on the liposomes or directly to the lipid component of the liposomes. Suitable compounds are polyethylene glycol (PEG) or hydroxyethyl starch (HES).

For the purposes of the present invention, the "active agent" or "drug" is Griffithsin (abbreviation GRFT). Whenever the term "Griffithsin" is used, it also encompasses derivatives or variants of Griffithsin. Griffithsin is a carbohydrate-binding agent (CBA) and belongs to the group of mannose-specific natural lectins from *Griffithsia* sp. The ability of CBAs to strongly bind mannose or fucose (e.g. prominently displayed by SARS-CoV spike like glycoprotein S) is thought to therapeutically interfere with the virus-cell fusion process. Depending on the pH, CBAs exist either as dimers or tetramers. Tetramers of plant lectins are typically -0.5 Å (5 nm) in diameter. Thus, many CBAs can be entrapped in, and delivered from, a liposome of a diameter of ∼100-200 nm to agglutinate intracellular viral reservoirs.

Suitable "Griffithsin derivatives or variants" are for example described in WO 2016/130628 A1. These encompass Griffithsin mutant polypeptides which contain additional insertions, deletions, substitutions or additions relative to the wild-type polypeptide. They encompass also Griffithsin dimers or multimers. The Griffithsin derivatives or variants can be obtained by, for instance, glycoylation, amidation, carboxylation, phosphorylation or by the creation of addition salts, amides, esters or N-acyl derivatives. The Griffithsin derivatives or variants also encompass fusion proteins of Griffithsin coupled to an effector component, e.g. PEG, dextran, albumin, an immunological reagent, a toxin or a solid support matrix.

In other embodiments of the invention, the active agent complex may also comprise one or more antiviral drugs or virucidal or virostatic agents (such as an interferon), nucleoside analogs, or non-nucleoside anti-viral drugs. Examples include indinavir (a.k.a. Crixivan^{®}, Merck & Co., Inc., Rahway, NJ; saquinavir (*N*-tert-butyl-decahydro-2-[2(R)-hydroxy-4-phenyl-3(S)-[[*N*-(2-quinolylcarbonyl)-L-asparaginyl]-amino]butyl]-(4aS,8aS)-isoquinoline-3(S)-carboxamide; MW 670.86 Da (a.k.a. Fortovase^{®}, Roche Laboratories, Inc., Nutley, NJ); or nelfinavir (i.e., nelfinavir mesylate, a.k.a. Viracept^{®}. Other examples of antiviral drugs include reverse transcriptase inhibitors, such as tenofovir disoproxil fumarate (9-[(R)-2-[[bis[[(isopropoxycarbonyl)oxy]methoxy] phosphinyl]methoxy] propyl]adenine fumarate (1:1); MW = 635.52 Da; a.k.a. Viread^{®}, Gilead Sciences, Foster City, CA, USA), 2-Ethylbutyl-(2S)-2-{[(S)-{[(2R,3S,4R,5R)-5-(4-aminopyrrolo[2,1-f][1,2,4]triazin-7-yl)-5-cyan-3,4-dihydroxytetrahydro-2-furanyl]methoxy}(phenoxy)phosphoryl]amino} a.k.a. Remdesivir^{®}, Gilead Sciences, Foster City, CA, USA), Antiviral drugs can also be virus-specific siRNA, antisense or sense DNA molecules. Preferably, the additional antiviral drugs are broad spectrum antiviral drugs.

In another embodiment of the invention, the active agent complex further comprises one or more accessory factors comprising bivalent cations, co-enzymes, enzyme activators, or pH-modifying agents.

In a particular preferred embodiment of the present invention, the nanocarriers are phosphatidylcholine-derived liposomes labeled on their surface with Fuc-4-Chol and containing the Griffithsin or Griffithsin derivatives as an encapsulated drug.

The antiviral activity of Griffithsin has been reported e.g. in US 7,884,178 B2 or US 8,394,764 B2. It recognizes a conserved glycoprotein structure on the surface of a number of retroviruses (HIV-1, SIV, HTLV, FIV, MLV, BLV, equine infectious virus) and non-retroviruses: HCV, influenza viruses (including influenza A, A/H5N1 or A/H7N9) or corona viruses (including SARS-CoV-1 and SARS-CoV-2).

Non-encapsulated Griffithsin has a broad mechanism of action including inhibition of viral fusion, entry, assembly and release. However, it is not able to enter infected cells to a notable degree. The inventors therefore focus on the encapsulated version of this CBA designed to eliminate intracellular viral reservoirs or propagating pathogens. As mentioned above, in a particular preferred embodiment the treatment version of the present invention comprises both encapsulated as well as non-encapsulated Griffithsin as a combination. The ratio of encapsulated to non-encapsulated Griffithsin is between 10:1 to 1:10, preferably 10:1, 5:1, 2:1, 1:1, 1:2, 1:5, or 1:10. In the combination of the present invention where encapsulated Griffithsin is combined with free Griffithsin it is possible to attack the virus both intracellularly and extracellularly. The treatment using such a combined present invention for the first time may enable to attack intracellular and extracellular viral reservoirs. It thus has the principal potential to enable functional or even eradication cures of respiratory viral infections.

The encapsulation of Griffithsin enables to use much less Griffithsin than would be necessary for the unencapsulated administration. Thus, it is advantageous as it decreases costs, increases efficacy, facilitates treatment schemes and reduces side effects in the patient.

The active agent complex or pharmaceutical composition comprising encapsulated GRFT is suitable as a prophylactic treatment preceding viral infection or as a therapeutic treatment during viral infection or as a post-exposure prophylaxis following viral infection. A particular preferred embodiment is a combination of (a) CLR-TS-nanocarriers containing Griffithsin with (b) non-encapsulated Griffithsin as a prophylactic treatment preceding viral infection or as a therapeutic treatment during viral infection or as a post-exposure prophylaxis following viral infection.

In some prophylactic applications, the active agent complex, pharmaceutical composition or combination of the present invention is administered to a subject at risk for exposure to the virus. In some therapeutic applications, it is administered immediately to a subject exposed to the virus. In some therapeutic applications, it is administered to a subject after exposure to virus where viral infection progresses to a clinical disease. In some embodiments, prophylactic compositions prevent or reduce the risk of viral infection. Such an amount is defined to be a prophylactically effective amount or dose. In this use, the precise amounts also depend on the patient's state of health, weight, and the like. When used in patients, effective amounts for this use will depend on the severity and course of the disease, disorder or condition, previous therapy, the patient's health status and response to the drugs, and the judgment of the treating physician.

In some therapeutic applications, the active agent complex, pharmaceutical composition or combination is administered to a patient already suffering from the viral infection, in an amount sufficient to cure or at least partially arrest at least one of the symptoms of the disease or condition. Amounts effective for this use depend on the severity and course of the disease or condition, previous therapy, the patient's health status, weight, and response to the drugs, and the judgment of the treating physician. Therapeutically effective amounts are optionally determined by methods including, but not limited to, a dose escalation and/or dose ranging clinical trial.

This treatment concept is using the CLR entry pathway and is independent of ACE2 receptor, which has been shown to be down regulated after SARS-Covid19 viral entry (Tai et al., Characterization of the receptor-binding domain (RBD) of 2019 novel coronavirus: implication for development of RBD protein as a viral attachment inhibitor and vaccine, Cellular & Molecular Immunology, 2020; Hoffmann et al., SARS-CoV-2 Cell Entry Dependson ACE2 and TMPRSS2 and Is Blocked by a Clinically Proven Protease Inhibitor, Cell, 2020). The advantage of employing the combination of the present invention in therapeutic application is that recruited immune cells that would be prone to get infected with viruses are prevented from being infected. In addition, CLR-ligand (a frucose polysaccharide) binds predominantly to the DC-SIGN and CD209L of the CLR family. Altogether, viral binding to host cell surface is prevented, viral entry is inhibited, and viral replication is inhibited through intracellular GRFT uptake and agglutination.

CLRs function as 'pathogen entry ports' into a cell expressing such receptors. As to their biological function, the endocytic members of APC-expressed CLRs serve for the uptake of pathogens and their intracellular break-down, subsequent controlled processing of pathogen-derived protein antigens to 10-mer or 12-mer peptides and the association of such antigens with class I or class II proteins of the APC's major histocompatibility complex (MHC). These combinatorial molecules allow for MHC-restricted - i.e., strongly controlled - presentation of the antigens on the APC surface where instructing, in a delicately balanced concert with other surface molecules and soluble signals, other immune cells to elicit a protective cellular and/or humoral immune response against the pathogen initially internalized. Importantly, various pathogens have evolved to resist intracellular degradation by APCs.

The design of the nanocarriers of the present invention (known as TargoSphere^{®} and distributed by the applicant) took advantage of a crucial cellular uptake mechanism for infectious agents that depends on their surface decoration with certain distinctive carbohydrates. Also, as the nanocarriers of the present invention can be scaled at diameters of between approx. 50-200 nm - hence falling in the nanocarrier category - they are sized in the range of most viruses. Like pathogens, the nanocarriers of the present invention are internalized in clathrin-dependent manner whereupon both carrier and payload (i.e. Griffithsin) are delivered into endosomes. Furthermore, and again like reservoir-forming pathogens, a given therapeutic payload can escape the endo(lyso)somal compartment to access the cytoplasm.

The nanocarriers, thus, enable a multitude of applications, which makes this technology a platform to address many disease indications. Finally, this potential is due to the crucial role of APCs in the induction, direction and termination of protective antigen-specific immune responses and the maintenance of immunotolerance.

Therefore, some key benefits and potentials of the nanocarriers of the present invention are:
- To selectively address all cells with CBD known as major pathogen reservoir or replication sites;
- To enable targeted delivery of drugs interfering with trans-cellular infection from pathogen reservoirs residing within myeloid APCs;
- To enable targeted delivery of drugs interfering with active intracellular pathogen propagation;
- To enable targeted delivery of specific compounds into APCs -specifically mDCs - for inducing or enhancing protective primary immune responses for active protective or therapeutic effect;
- To significantly reduce or abrogate drug- or vaccine-related toxic effects on non-target cells; and
- To protect the entrapped active agents from premature systemic degradation.

The particular preferred combination of the present invention provides a superior effect over both the encapsulated active agent alone and the non-encapsulated agent alone. Superior effect means superior efficacy, superior safety / toxicity profile and/or superior drug availability.

### 6. EXAMPLES

### Essential Definitions:

- 'TargoSphere'^{®} or 'TargoSpheres' (TS) for a ligand-targeted nanocarrier(s) developed and marketed by Rodos Biotarget GmbH, Hannover, Germany, later acquired by SolmicBiotech GmbH, Düsseldorf, Germany and further marketed by the affiliated companies Bioloving Germany Biotech GmbH & Co KG and Biolife Holding AG, both Heidelberg, Germany.
- 'CLR-TargoSphere or CLR-TS for a lipid/carbohydrate-based TargoSphere variant that allows to specifically address CLR- and CRD-positive cells, and that enables to encapsulate Griffithsin;
- RBT05 is the company's pipeline designation of CLR-TargoSphere and Griffithsin (also GRFT)

### Example 1

### Fucose derivative specific for C-type lectin receptors (CLRs): Synthesis of Fucose Targeting Ligand at Laboratory Scale

The production of a fucose derivative specific for CLRs and CRDs - also briefly termed 'Fuc-C4-chol targeting ligand' - was principally based on Kawakami et al. and Nishikawa et al. [Kawakami S et al. Asialoglycoprotein receptor-mediated gene transfer using novel galactosylated cationic liposomes. Biochem Biophys Res Commun 1998; 252:78-83, and Nishikawa M et al. Glycosylated cationic liposomes for carbohydrate receptor-mediated gene transfer. Methods Enzymol 2003; 373:384-99], with modifications, as described in WO 05/092288 A1. Importantly, this synthesis protocol was modified with the objective to improve the purity of targeting ligands from by-products generated in the process. Accordingly, Steps 1, 2, 4 and 5 of below described ligand synthesis were improved.

Step 1: Acetylation of fucose and synthesis of 1,2,3,4-tetra-O-acetyl-fucopyranoside: 152 mmol of fucose was added stepwise under continuous agitation to a mixture of 175 mL acetic anhydride and 1.25 mL perchloric acid pre-cooled on ice. The solution was then stirred at RT for a further 3 h under the exclusion of air humidity. This reaction mixture was poured onto 200 mL of iced water, before adding 300 mL of ethyl acetate. After phase separation in a funnel, the collected organic layer was washed x3 with 100 mL of cold water and dried over anhydrous magnesium sulfate. Filtration and evaporation of the solvent resulted in 1,2,3,4-tetra-O-acetyl-fucopyranoside as an oil. 25 g L-fucose (150.49 mmol, 97% pure ACROS) were suspended in 160 mL of pyridine. The suspension was treated with 80 mL of acetic anhydride and stirred overnight. The reaction mixture was poured onto iced water and extracted with 200 mL of CH₂Cl₂. The organic phase was washed x3 with 100 mL of cold 4 M HCl, x3 with 100 mL of saturated aqueous NaHCO₃ solution and dried over MgSO₄. The solvent was evaporated to yield 1,2,3,4-tetra-O-acetylfucopyranose as a white solid.

Step 2: Synthesis of 1-bromo-2,3,4-tri-O-acetyl-fucopyranoside: 47.6 mmol of 1,2,3,4-tetra-O-acetyl-fucopyranoside were added slowly (step wise) to 75 mL of a hydrogen bromide/acetic acid solution (33%) while stirring in an ice bath; this mixture was further stirred overnight at 4°C. After phase separation, the organic layer was collected, and the aqueous layer was washed x3 with 100 mL of ethyl acetate. Combined ethyl acetate fractions were subsequently neutralized by adding 200 ml of a saturated sodium bicarbonate solution. The neutralized organic phase was washed with 200 mL of a 1 % sodium chloride solution, resulting layers were separated, and the organic layer was dried with magnesium sulfate, filtered, and evaporated to give 2,3,4-tri-O-acetyl-fucopyranosylbromide.

Step 3: Synthesis of 2-S-(2,3,4-tri-O-acetyl-thiofucopyranosyl)-2-thiopseudourea hydrobromide: According to Wolfrom and Thompson [Wolfrom ML, Thompson A. Acetylation. In: Whistler RL, Wolfrom ML (eds.). Methods in Carbohydrate Chemistry, Vol. II: Reactions of Carbohydrates. Academic Press, New York 1963: pp. 211-5], 45.17 mmol of dried 1-bromo-2,3,4-tri-O-acetyl-fucopyranoside was dissolved in 20 mL of dry acetone. The solution was then transferred to a 250-mL round-bottomed flask, and 50 mmol of thiourea were added under an argon atmosphere. The reaction mixture was refluxed for 15 min and allowed to cool off in an ice bath, whereupon precipitated 2-S-(2,3,4-tri-O-acetyl-thiofucopyranosyl)-2-thiopseudourea hydrobromide was collected by filtration.

Step 4: Synthesis of cyanomethyl-2,3,4-tri-O-acetyl-thiofucopyranoside: Under continuous agitation, 12.3 mmol of 2-S-(2,3,4-tri-O-acetyl-thiofucopyranosyl)-2-thiopseudourea hydrobromide were dissolved in 3.1 mL of chloroacetonitrile plus 24 ml of 1:1 (v/v) water/acetone. Upon obtaining a nearly clear solution, 14.3 mmol potassium carbonate and 24.9 mmol sodium bisulfate were added, and the reaction mixture was stirred for 30 min at room temperature (RT). For purification, this mixture was evaporated, and the residue was dissolved in ethyl acetate. The product, cyanomethyl-2,3,4-tri-O-acetyl-thiofucopyranoside, was subsequently purified by the patented procedure described above to lead to crystallization of the intermediate product.

Step 5: Deacetylation and synthesis of 2-imino-2-methoxyethyl-1-thiofucopyranoside (IME-thiofucoside): Five mmol of cyanomethyl-2,3,4-tri-O-acetyl-thiofucopyranoside were dissolved in 18 mL of methanol and 2 mL of 0.1 M sodium methoxide and kept at RT overnight. Then, 103.66 mmol of cyanomethyl-2,3,4-tri-O-acetyl-thiofucopyranoside were dissolved in 350 mL of methanol and 41.5 mL of 0.01 M sodium methoxide and kept at RT overnight. After methanol evaporation, the resulting IME-thiofucoside-containing syrup was further used for coupling this intermediate to N-(4-aminobutyl)-(cholesten-5-yloxyl)formamide (see Step 6, Part 2).

Step 6: Synthesis of cholesten-5-yloxyl-N-(4-((1-imino-c-b-D-thiofucosylethyl)amino)butyl) formamide: Part 1: 5.22 mmol N-boc-1,4-butane diamine and 4.75 mmol cholesteryl chloroformate were combined in 95 mL of chloroform and stirred for 24 h at RT. A solution of 9.5 mL trifluoroacetic acid in 24 mL chloroform was added dropwise, and the reaction mixture was stirred for 4 h at 4°C. Chloroform evaporation and removal of trifluoroacetic acid by co-evaporation with 100 mL of toluene gave the reactive cholesterol derivative, N-(4-aminobutyl)-(cholesten-5-yloxyl)formamide, which was crystallized by adding 15 mL of n-pentane. Part 2: 2.5 mmol of the reactive fucose derivative, IME-thiofucoside, were dissolved in 20 mL of dry pyridine and added 153 mL of triethylamine. The final reaction mixture was obtained by subsequent addition of 1 mmol of the crystallized cholesterol derivative and was kept at RT for 24 h. Following evaporation of the solvent and co-evaporation with toluene, the material was suspended in 30 mL of distilled water, dialyzed for 2 days at 4°C against water in a dialysis tube with a 12-kDa cut-off, then lyophilized, and finally washed with diethylether. The resulting cholesten-5-yloxyl-N-(4-((1-iminoc-β-D-thiofucosylethyl)amino)butyl) formamide was further employed as a CLR-specific targeting ligand.

General remarks:
- The derivative cholesten-5-yloxyl-N-(4-((1-imino-c-b-D-thiofucosylethyl)amino)butyl) formamide is subsequently referred to as 'Fuc-C4-Chol targeting ligand', 'FC ligand' or 'targeting ligand', respectively.
- Throughout synthesis, intermediates were structurally confirmed by nuclear magnetic resonance spectroscopy. The final product was purified by column chromatography.

### Example 2

### Formulation of GRFT-loaded CLR-TargoSpheres

In the following experiments the CBA (lectin) Griffithsin which has a considerable antiviral potential was used. In its non-encapsulated form, it is active at very low concentrations. This CBA recognizes a conserved glycoprotein structure on the surface of a number of viruses (e.g. influenza A viruses (e.g. H5N1) or corona viruses (including SARS-CoV)).

Non-encapsulated GRFT has a broad mechanism of action including inhibiting viral fusion, entry, assembly and release. However, it is not able to enter infected cells to a notable degree. The inventors therefore focus on the TargoSphere-encapsulated version of this CBA designed to eliminate intracellular viral reservoirs. Besides, the applicant's exploratory treatment version comprises both TargoSphere-encapsulated as well as non-encapsulated CBA. It is thus envisioned to develop the combination as a broad-spectrum antiviral treatment that will address a range of viral species both in their intracellular and their extracellular locations. By designing treatment with such a combination may for the first time enable to attack intracellular and extracellular viral reservoirs. It thus has the principal potential to enable functional or even eradication cures of viral infections.

GRFT was successfully encapsulated into TargoSpheres. Specifically, TargoSpheres were prepared that encapsulated GRFT only, as well as TargoSpheres encasing GRFT plus fluorescein-isothiocyanate (FITC)-conjugated dextran.

The formulation of TargoSpheres encapsulated with GRFT- or GRFT/dextran x FITC were achieved by reversed phase preparation with the complete TargoSphere membrane lipid mixture as well as targeting ligand dissolved in chloroform under vacuum. While the organic dissolvent was thus evaporated, TargoSphere/lipid/ligand complexes were retained as an oily film. Regular air pressure was re-established, and GRFT and/or dextran x FITC were added in aqueous solution, furthermore adding ether as a dissolvent. After a final round of high pressure encapsulation at 5.65 bar (82 psi), ether removal led to the production of GRFT- or GRFT/dextran x FITC-loaded TargoSpheres in PBS, while non-encapsulated GRFT was removed.

The characteristics of the resulting preparations were:
1. CLR-TargoSpheres loaded with GRFT: Average diameter (ZAve) = -142 nm.
2. CLR-TargoSpheres loaded with 9:1 GRFT:dextran x FITC: ZAve = -147 nm.

In all cases, TargoSpheres revealed an uptake efficiency of 9.4% of the initial GRFT or GRFT/dextran x FITC concentrations of 2 mg/ml, each. Hence, 1 mL of TargoSpheres contained final concentrations of approx. 188 µg of the encapsulated agent or agents.

### Example 3

### Effect of CLR-TargoSphere encapsulated GRFT formulations in Vero6 cell viability

VeroE6 cells (5000 cells per well of 96 well plate) were grown in Minimum Essential Medium with Hanks' and Earle's salts supplemented with 10% fetal bovine serum and non-essential amino acids in a humidified CO₂ incubator. After 6-8 hours of plating them into the wells, cells were adhered to the bottom of the wells. Media was then removed from the wells, washed with PBS and fresh media was added into them. Later cells with or without virus infection were treated with unencapsulated Griffithsin (GRFT), C-type lectin receptor (CLR)-TargoSphere^{®} encapsulated GRFT (CLR-TS-GRFT) and Ripamycin. After 3 days of post-infection and/or treatment, cell viability was measured using Cell Titer 96^{®} AQueous One Solution Cell Proliferation Assay (MTS). For infection of the cells, SARS-CoV-2 strain Muc-IMB-1 or SARS-CoV-2 strain Muc-IMB-CB (Source for Virus: German Armed Forces Institute of Microbiology, Munich, Germany) were used in the study. All handling of SARS-CoV-2 was conducted under BSL-3 conditions in accordance with biosafety guidelines. CLR-TS-GRFT formulations were non-GMP and produced by the applicant using Standard Operating Procedures (SOPs).

### Results:

Treatment of VeroE6 cells with CLR-TargoSphere encapsulated GRFT formulations and unencapsulated GRFT showed no specific toxic effect on the cells compared to a mock control. Treatment of cells with approved Rapamycin which was suggested to include in COVID-19 emergency treatment slightly decreased the viability of cells. The positive control, SARS-CoV-2 strain Muc-IMB-1 infected cells showed a significant decrease in the viability of cells as expected compared to a mock control **(****Figure 1****).**

Further, treatment of cells harbouring SARS-CoV-2 strain Muc-IMB-1 **(****Figure 2****)** or SARS-CoV-2 strain Muc-IMB-CB **(****Figure 3****)** with the formulations according to the present invention improved viability of cells compared to unencapsulated GRFT treated cells or virus infected cells after 3 days of post infection.

Thus, CLR-TS encapsulated GFRT formulations show
1. No toxicity in VeroE6 cells.
2. Higher efficacy than unencapsulated GRFT and approved Rapamycin by protecting cells from SARS-CoV-2 strain Muc-IMB-1 and SARS-CoV-2 strain Muc-IMB-CB.

### Example 4

### Targeting of Influenza A virus H1N1

- Host cells: Human A549 lung epithelial cells, i.e. the type of cell typically infected in patients and expressing C-type lectin receptors
- Infectious IAV H1N1: were infected w/ the IAV H1N1v strain A/Regensburg/D6/09.
- Potential of the test agents to inhibit IAV propagation: In the infection model employing the host cells and the IAV H1N1v strain, intracellular inhibition of viral propagation by RBT-05 (CLR-TS w/ GRFT + free GRFT), by non-targeted nanocarrier encapsulated plus free GRFT, and by non-encapsulated GRFT was tested in serial dilutions of 1:2 to 1:100 against untreated infection conditions. The read-out was performed by determining virus-induced plaque-forming units (PFUs) relating to the actual viral copy numbers (n = 3 per reading point). Data were normalized and set at 100% for the non-treated infection control (c.f. **Figure 4****).**

### RESULTS:

Untreated controls showed a mean of about 6.52 log₁₀ PFUs/mL or about 76,000,000 viral copies/mL. The detection threshold of the assay was about 1.7 log₁₀ PFUs/mL or about 50 viral copies/mL.

All numerical data provided below are to be understood relative to untreated controls.
- RBT-05: Treatment showed a concentration-dependent PFU reduction and reached the detection limit at 1:10 dilution. (However, the actual dilution may be lower as the next dilution tested was 1:100.)
- Non-targeted nanocarrier w/encapsulated GRFT + free GRFT: Treatment showed a concentration-dependent PFU reduction and reached the threshold at 1:1 dilution.
- Non-encapsulated GRFT: Treatment showed a concentration-dependent PFU reduction, but did not reach the detection limit.

The curve shapes of the three conditions differed from one another.

### Example 5

### Organ Distribution

The total 12 wild-type mice were administered with representative Zr-89-oxine-TargoSphere: 4 animals intravenously (IV), 4 animals subcutaneously (SC) and 4 animals intranasally (IN) were administered with the compound. In each group, 2 animals received CLR+ and 2 animals received CLR- variants of TargoSphere. After the first hour PET-acquisitions, 24h hour PET-scans were taken and then the animals were euthanized, and the organ activities measured.

As a result, it was seen that in the SC and IN cases there was very low leakage, migration, redistribution ratio from the injection points. Most of the activity remained at these points for 24 h. A moderated activity was detected in the selected lymph nodes but no other remarkable organ accumulation was seen on the scans. The 1 h scans of the IV applications showed massive liver and spleen uptakes, the lung activity was a bit higher in the first moments (until 10-20 min) than earlier. After 24 h scanning, the liver and spleen activities are still high, and most of the injected activities remain there. The 24 h cumulative isolated liver and spleen activities of the CLR+ variants were higher (avg. 80% of total injected activity) than of the CLR- values (avg. 54%) while the avg. isolated kidney uptakes show the opposite (3% vs. 12%).

### Example 6

### Intranasal Application

4 times 6 µl of 68Ga-TS were administered intranasally into each animal (Animals 19 and 20) in 5 minutes. No irritation could be observed by this injection amount, while the injected activities (2-5 MBq / 24 µl) proved to be enough for proper PET tracking. 50 minutes dynamic scans showed that most of the activity (>95%) remained on the nasal mucosa over the entire 90 min scanning time but a lower and clearly visible activity part reached the trachea tract and the lungs right in the first minutes. The entered particles accumulated in these tracts without releasing the activity into the systemic circulation over the next 50 min. Only a moderated activity part appears in the blood circulation, and then in the urinary tract in the first 50 min. The scans revealed very slow mucous and pulmonary activity release that again implied the particles' high degree of integrity and labeling stability in the first 60 min post injection. The 90 min static images show the same distribution and the retrieved fused PET/CT could clearly prove a definite lung uptake.

Selected organs were collected after the last 90 min. static PET acquisitions. Results are summarized I.D./g values in the below Table 1.

**Table 1: Ex vivo results of intranasal (I.N.) administered ⁶⁸Ga-Targosphere 90 p.l.**

| **Organ** | **Animal No. 19** | **Animal No. 20** |
|---|---|---|
| Liver | 0.03% | 0.05% |
| Spleen | 0.04% | 0.07% |
| Kidneys | 0.05% | 0.07% |
| Lymph Node | 0.10% | 0.13% |
| Urinary Bladder | 0.37% | 0.70% |
| Salvary Gland | 0.08% | 0.16% |
| Blood | 0.13% | 0.21% |
| Brain | 0.06% | 0.08% |
| Lungs | 1.77% | 0.92% |

These results clearly show that the intranasal application leads to the desired accumulation in the lung.

## Claims

1. An active agent complex comprising Griffithsin (GRFT) as an active agent encapsulated in a lipid-based or polymer-based nanocarrier and at least one cell-specific targeting ligand on the surface of the nanocarrier, wherein the targeting ligand specifically binds to a receptor on the surface of a CRD-positive or CLR-positive cell infected with, or susceptible to infection with, a respiratory virus,
for use in the treatment or prevention of an infection caused by a respiratory virus.

2. The active agent complex for the use of claim 1, wherein the targeting ligand is a small chemical compound, an antibody, an antibody fragment, a sugar, a peptide or an aptamer.

3. The active agent complex for the use of claim 1, comprising the Griffithsin encapsulated in a liposome and at least one carbohydrate or derivative thereof as a targeting ligand on the outer surface of the liposome, wherein the targeting ligand specifically binds to a receptor expressing C-type lectin-like carbohydrate recognition domains (CRD) or other receptors with carbohydrate recognition domains on the surface of the cell.

4. The active agent complex for the use of any of claims 1 to 3, wherein the CRD-positive cell or CLR-positive cell is selected from airway epithelial cells, endothelial cells of the lymphatic or blood system, and immune cells.

5. The active agent complex for the use of claim 4, wherein the immune cells are bone marrow-resident myeloid progenitor cells, monocytes, myeloid dendritic cells, macrophages, follicular dendritic cells, plasmacytoid dendritic cells, T lymphocytes and natural killer cells.

6. The active agent complex for the use of any of claims 1-5, wherein the respiratory virus is a respiratory syncytial virus, influenza virus, parainfluenza virus, metapneumovirus, rhinovirus, adenovirus, bocavirus or coronavirus.

7. The active agent complex for the use of claim 6, wherein the coronavirus is SARS-CoV-1, MERS-CoV or SARS-CoV-2.

8. The active agent complex for the use of claim 6, wherein the influenza virus is Influenza Virus A, B or C or a mixed human-animal type.

9. The active agent complex for the use of any of claims 1-8, wherein the active agent complex further comprises one or more accessory factors comprising bivalent cations, co-enzymes, enzyme activators, or pH-modifying agents.

10. The active agent complex for the use of any of claims 1-9, wherein it is administered by injection through a transvascular route, a subcutaneous route, an intradermal route, a bone-marrow-directed route, an intrapulmonary route, an intranasal route, an intraocular route, an intraperitoneal route or an intravenous route.

11. The active agent complex for the use of any of claims 1-10, wherein the targeting ligand is monofucose, polyfucose, cholesten-5-yloxy-N-(4-((1-imino-2-D-thiofucosylethyl)amino)alkyl)formamide (Fuc-C4-chol) or cholesteryl-(4-(3-((2R,3S,4R,5S,6S)-3,4,5-trihydroxy-6-(hydroxymethyl)tetrahydro-2H-pyran-2-yl)thioureido)butyl)carbamate (Thiourea-Galac-C4-chol).

12. A pharmaceutical composition comprising the active agent complex as defined in any of claims 1-11 and a pharmaceutically acceptable carrier for use in method of treating or preventing an infection caused by a respiratory virus.

13. A pharmaceutical combination comprising (a) the active agent complex as defined in any of claims 1-11 and (b) non-encapsulated Griffithsin for use in a method of treating or preventing of an infection caused by a respiratory virus.

14. The pharmaceutical combination for the use of claim 13, wherein the ratio of (a) to (b) is 10:1 to 1:10.
